# EUROPEAN PATENT SPECIFICATION

(11) **EP 1 397 176 B1**
(45) Date of publication and mention of the grant of the patent: **14.03.2007**
(21) Application number: 01956493.9
(22) Date of filing: 21.06.2001
(51) Int. Cl.: A61M 25/01, A61B 8/12

(54) **APPARATUS FOR CROSSING A HEART VALVE**
GERÄT ZUM DURCHDRINGEN EINER HERZKLAPPENÖFFNUNG
DISPOSITIFS DESTINES A TRAVERSER UNE VALVULE

(43) Date of publication of application: 17.03.2004
(73) Proprietor: Abbott Laboratories Vascular Enterprises Limited, Dublin 2 (IE)
(72) Inventor: HAASE, Karl, 67346 Speyer (DE); SCHWAGER, Michael, CH-8404 Winterthur (CH)
(74) Representative: Schmitz, Hans-Werner
(86) International application number: PCT/EP2001/007035
(87) International publication number: WO 2003/000331

(56) References cited:
- EP-A- 0 940 123
- WO-A-00/65987
- WO-A-94/10907
- US-A- 4 748 986
- US-A- 4 832 047
- US-A- 5 402 799

## Description

### FIELD OF THE INVENTION

The present invention relates to a guide wire according to the preamble part of claim 1. Such a guide wire is known from US 4,748,986.

Moreover, US 4,832,047 and WO00/65987 disclose further examples of a generic guide wire.

### BACKGROUND OF THE INVENTION

The human heart comprises four valves, which are located at the exit of each of the four chambers of the heart. These are the aortic valve, pulmonary valve, mitral valve, and tricuspid valve. The valves are made up of thin, very strong flaps of tissue, or leaflets, that open and dose as the heart pumps. Through precisely synchronized opening and closure, these valves act as gates that keep blood flowing in the proper direction and regulate blood flow through the chambers of the heart and throughout a person's body. In some people, rheumatic fever, congenital heart defects, the aging process, or other factors, may cause sticking, hardening in the form of calcification, and/or narrowing in the form of stenosis of one or more of the heart valves that causes the affected valves to open inadequately. Alternatively, valvular insufficiency or regurgitation may occur, wherein a valve closes inadequately and allows blood to flow backwards, or leak, through the valve. Both insufficient and stenosed valves may require intervention.

Defective heart valves may either be repaired or replaced. Valve replacement involves removal of the affected valve and replacement with either a mechanical valve, typically metal, or with a preserved biological valve, typically made from pig or cow tissue. Replacement procedures have several drawbacks. They are usually highly invasive with substantial potential complications and long recovery times, and they normally entail stopping the heart and placing the patient on cardiopulmonary bypass. Additionally, biological valves tend to wear and degrade over time, thus limiting their useful life to about 10 years. Implantation of a metal mechanical valve, meanwhile, requires a patient to take powerful blood thinning medication, such as Coumadin, for the remainder of his or her life. Defective valves have also been replaced with homografts, which are preserved valves that have been harvested from human donors, and via the Ross procedure, in which the patient's own pulmonary valve is transplanted to the aortic position and a pulmonary homograft is placed in the pulmonary position.

When possible, repair of defective valves is generally preferred to replacement. Valvular regurgitation often may be repaired with a ring placed in the orifice that the leaky valve covers, to reduce the area of the orifice and allow the valve to cover it more effectively. Alternatively, a surgeon may carefully remodel the size and shape of the leaflets. Both techniques provide improved heart muscle performance, since the papillary muscles and supporting chordae tendinae are left intact. Additionally, saving the person's native valve may decrease the number of repeated surgical procedures required and the need for long term anticoagulation medication.

Repair of stenosed valves is commonly indicated for patients with narrowed valves. Commisurotomy entails separation of the leaflets of the stenosed valve, which have fused together at their commissures, or points of contact. Many candidates for commisurotomy are today treated by a newer approach to stenotic valve repair known as valvuloplasty. A primary advantage of valvuloplasty procedures is that they are performed in a minimally invasive fashion. The patient's chest is not surgically opened, and the patient's heart need not be temporarily stopped.

Valvuloplasty is performed in a manner similar to balloon coronary artery angioplasty. A catheter having a deflated expandable balloon at its distal end is inserted into an artery or vein, usually in the leg, under a local anaesthetic and conscious sedation. Under angiographic/X-ray/fluoroscopic visualization, this catheter is advanced into the heart and positioned at the site of the damaged valve across the narrowing. A dye is injected to facilitate imaging of the heart, as well as to locate the damaged valve during advancement of the catheter, and measurements are made to determine the severity of the valve narrowing. If deemed necessary, the balloon is expanded to widen the valve, which improves flow through the valve. The balloon may be expanded and collapsed several times to stretch the hardened valve. Repeat heart measurements are taken to ensure that valve function has improved. The catheter is then removed from the patient.

Valvuloplasty procedures also may be performed transseptally. In transseptal procedures, a cardiologist creates a tiny hole in the wall between the chambers of the heart. The balloon catheter is then advanced through the hole and positioned so the balloon is directly inside the narrowed valve. The balloon then is inflated and deflated one or more times to widen the valve opening. Once the cardiologist has determined that the opening of the valve has been sufficiently widened, the balloon is deflated and removed.

Clearly, a variety of treatment options exist for patient's suffering from valvular insufficiency and valvular stenosis. Proper diagnosis of the cause, type, and severity of defects is imperative to ensuring that an adequate treatment modality is selected. A common diagnostic technique entails advancement of a catheter or sensors through the affected valve into the heart so that images and/or measurements, such as pressure or flow velocity, may be taken. A guide wire is usually advanced through the valve first, and then the catheter/sensors are advanced over the guide wire, A similar technique is used during valvuloplasty. Guide wires with built-in diagnostic sensors, such as the WaveWire and FloWire products distributed by JOMED, Inc., of Rancho Cordova, CA, are also available. The WaveWire comprises a pressure sensor, while the FloWire comprises a flow velocity sensor.

Unfortunately, standard guide wires are generally not specifically designed for transvalvular procedures, i.e. procedures across a heart valve. Standard wires include both guide wires used for diagnostic purposes exclusively and percutaneous transluminal angioplasty ("PTA") wires for peripheral applications, as well as percutaneous transluminal coronary angioplasty ("PTCA") wires for coronary applications. Guide wires are normally cylindrical, are often coated with a hydrophilic substance, such as polytetrafluoroethylene ("Teflon"), to reduce friction during advancement, and typically comprise a stiff core wire connected to a flexible distal tip. The core wire and distal tip have different, complementary functions. The core wire provides "pushability", which is the ability to advance a wire through a patient's anatomy. The flexible distal tip provides "steerability" and "trackability", which are, respectively, the ability to route a wire through bifurcations in the patient's vasculature, and the ability for the wire to follow tortuous anatomy without kinking, perforating the vessel, or otherwise failing.

The flexible distal tip typically comprises a section of reduced diameter, as compared to the core wire, to increase flexibility. The section of reduced diameter commonly is covered with a coil so that the distal tip meets flush with the core wire. The coil, or a portion thereof, may be radiopaque to facilitate imaging of the tip, and thus positioning of the wire within a patient's vasculature.

The maximum outer diameter of standard guide wires generally ranges from about 0.35mm to about 0.51mm (0.014"-0.020"). These wires are normally about 180cm to 300cm in length, with the core wire extending the vast majority of that length. The flexible distal tips of PTA wires commonly extend about 6cm, while the flexible tips of PTCA wires often extend a greater distance of about 30cm. The distal tips are available in a variety of stiffnesses ranging from relatively floppy to relatively stiff, as compared to the core wires. These tips are tailored to a variety of intravascular applications.

For transvalvular procedures, guide wires having relatively stiff distal tips are most often used. The relatively stiff tips provide needed pushability that facilitates passage through a heart valve. However, a risk of myocardial perforation exists if these guide wires are over-inserted into the heart to a point where they contact an internal wall of the heart. Furthermore, when accessing the left ventricle via a retrograde approach through a stenosed aortic valve, trauma to the right or left coronary artery may occur. Coronary artery injury is most frequently associated with advancement of the guide wire into the coronary artery itself, potentially causing dissection, perforation, or abrupt vessel closure.

A further drawback of using standard guide wires for transvalvular procedures is that, especially at the outlet of stenosed valves, blood velocity may be quite high due to the decreased lumen through the valves. If the flexible distal tip of the guide wire is too floppy, the increased velocity may deflect the wire and make it difficult or impractical to accurately position the wire in the blood jet for passage through the valve. This, in turn, may make it impractical to advance a catheter and/or sensors over the guide wire and through the valve to evaluate the severity of transstenotic pressure loss, to determine if the patient is a candidate for valvular repair, or to perform valvuloplasty. Consequently, open surgery may be required for patients that otherwise would be candidates for less traumatic, minimally invasive therapy, such as valvuloplasty. Alternatively, if the flexible distal tip of the guide wire is too stiff, while the guide wire may eventually be passed through the valve, failed attempts at passage may damage or perforate leaflets of the valve and/or may generate dangerous emboli that are carried downstream by blood flow.

In view of the drawbacks associated with previously known methods and apparatus for crossing a heart valve, it would be desirable to provide an apparatus that overcome these drawbacks.

It would be desirable to provide an apparatus for crossing a heart valve that enable controlled positioning of a guide wire within a blood jet passing through the valve.

It would further be desirable to provide an apparatus that reduce a risk of myocardial perforation after crossing.

It still further would be desirable to provide an apparatus for crossing a heart valve that reduce a risk of damage to valve leaflets.

It would also be desirable to provide an apparatus that reduce a risk of emboli generation during crossing of a stenosed valve.

It would be desirable to provide an apparatus that reduce a risk of coronary artery trauma or dissection during crossing of an aortic valve.

It would be desirable to provide an apparatus that facilitate positioning of sensors and/or catheters across the valve.

### SUMMARY OF THE INVENTION

In view of the foregoing, it is an object of the present invention to provide an apparatus for crossing a heart valve that overcome drawbacks associated with previously-known an apparatus.

It is another object to provide an apparatus for crossing a heart valve that enable controlled positioning of a guide wire within a blood jet passing through the valve.

It is a further object to provide an apparatus that reduce a risk of myocardial perforation after crossing.

It is an object to provide an apparatus for crossing a heart valve that reduce a risk of damage to valve leaflets.

It is also an object to provide an apparatus that reduce a risk of emboli generation during crossing of a stenosed valve.

It is an object to provide an apparatus that reduce a risk of coronary artery trauma or dissection during crossing of an aortic valve.

It is an object to provide an apparatus that facilitate positioning of sensors and/or catheters across the valve.

These and other objects of the present invention are achieved by providing a specialized guide wire comprising a stiff core wire attached to a flexible distal tip. As compared to standard guide wires, guide wires of the present invention have an increased diameter and a shorter, relatively floppy distal tip. The increased diameter provides improved "pushability" to facilitate positioning in a blood jet and crossing of a heart valve. The short, relatively floppy distal tip is expected to reduce a risk of damage to valve leaflets during crossing, reduce a risk of coronary artery trauma or dissection, reduce a risk of emboli generation when the valve is stenosed, and reduce a risk of myocardial perforation after crossing the valve. Additionally, the decreased length of the distal tip facilitates positioning within the blood jet and limits deflection of the guide wire out of the jet.

In a preferred embodiment, the flexible distal tip comprises a section of rectangular, as opposed to circular, cross-section. The rectangular cross-section, in conjunction with the decreased tip length, is expected to cause the distal tip to vibrate, or "dance", when positioned within a blood jet at the outlet of a heart valve. This vibratory motion may be imaged and may be used to indicate when the distal tip is positioned within the blood jet. When positioned within the blood jet, the guide wire then may be advanced through the heart valve without damaging the leaflets of the valve or generating emboli. The distal tip is preferably radiopaque to facilitate imaging.

Guide wires of the present invention preferably have a maximum outer diameter of at least about 0.60mm (0.024"), and more preferably of at least about 0.69mm (0.027"). The flexible distal tips preferable have a longitudinal length that is less than about 5cm, and more preferably less than or equal to about 2.5cm. The guide wires preferably have a total longitudinal length of less than or equal to about 200cm, and more preferably of less than or equal to about 150cm. Optionally, diagnostic sensors may be attached to the guide wires.

Methods of using the apparatus of the present invention also are explained.

### BRIEF DESCRIPTION OF THE DRAWINGS

Further features of the invention, its nature and various advantages will be more apparent from the following detailed description of the preferred embodiments, taken in conjunction with the accompanying drawings, in which like reference numerals refer to like parts throughout, and in which:
FIGS. 1A-1C are, respectively, a schematic view of a guide wire of the present invention, a side view, partially in section, of the guide wire, and a cross-sectional view of the flexible distal tip of the guide wire along section line A-A in FIG. 1B; and
FIGS. 2A-2G are side views, partially in section, of the guide wire of FIGS. 1 disposed at a patient's stenosed aortic valve, depicting a method of using the apparatus in accordance with the present invention.

### DETAILED DESCRIPTION OF THE INVENTION

The present invention relates to an apparatus for crossing a heart valve. More specifically, the present invention relates to specialized guide wires adapted for more controlled passage through defective heart valves with reduced risk of leaflet damage, emboli generation, or myocardial perforation.

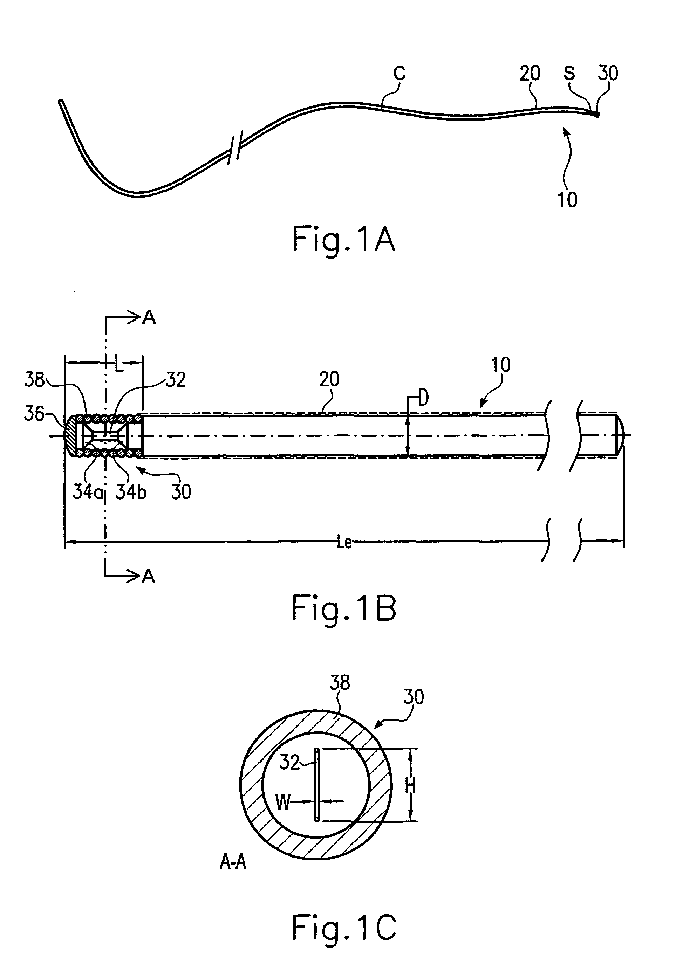

With reference to FIGS. 1, apparatus in accordance with the present invention is described. As seen in FIG. 1A, guide wire 10 comprises stiff core wire 20 connected to floppy distal tip 30. Guide wire 10 may be fabricated from a variety of materials and preferably comprises a stainless steel alloy. Guide wire 10 preferably is coated with hydrophilic coating **C**, which may comprise, for example, a polytetrafluoroethylene ("Teflon") coating. Coating **C** alternatively or additional may comprise a drug coating for localized, therapeutic drug delivery, or for combating thrombus formation around the wire. Guide wire 10 also may comprise one or more optional diagnostic sensors **S**, such as velocity, temperature, and/or pressure sensors. Optional sensor **S** illustratively is coupled to flexible distal tip 30 in FIG. 1A. but it should be understood that sensor **S** alternatively may be coupled anywhere along the length of guide wire 10.

Referring to FIG. 1B, core wire 20 and floppy distal tip 30 of guide wire 10 preferably are formed from a single wire that has been machined, for example, by laser cutting or by grinding, to the profile of guide wire 10. Alternatively, the core wire and distal tip may comprise separate sections that are joined using well-known techniques, Distal tip 30 also preferably is machined as one piece, but alternatively may be formed from multiple pieces.

Guide wire 10 has a longitudinal length Le, preferably less than or equal to about 200cm, and more preferably less than or equal to about 150cm. Floppy distal tip 30, meanwhile, has a longitudinal length I that is preferably less than about 5cm, and more preferably less than or equal to about 2.5cm. Length I is shorter that the length of the flexible distal tips of previously known standard guide wires, such as PTA and PTCA wires, which are commonly about 5cm to 30 cm in length. The shortness, as well as the flexibility, of distal tip 30 of the present invention is expected to reduce a risk of damage to leaflets of a heart valve when crossing the valve, to reduce a risk of emboli generation when the valve is stenosed, to reduce a risk of coronary artery trauma or dissection if wire 10 is inadvertently advanced into the coronaries, and to reduce a risk of myocardial perforation after crossing the valve.

Core wire 20 preferably is of circular cross section and has a maximum outer diameter **D** of at least about 0.60mm (0.024"), and more preferably of at least about 0.69mm (0.027"). Diameter **D** is significantly larger than the maximum outer diameter of many standard guide wires, which usually range between 0.35mm to about 0.51 mm (0.014"-0.020"). The increased diameter is expected to provide improved "pushability", and to facilitate positioning in a blood jet for crossing a heart valve.

Floppy distal tip 30 comprises section 32 of reduced cross-sectional area, Section 32 is connected on either side to transition sections 34a, 34b that taper from the cross-section of core wire 20 to the cross-section of section 32. Distal bulb 36, which has substantially the same cross-sectional area as core wire 20, is connected to transition section 34a. Floppy distal tip 30 is covered by coil 38, which extends between transition sections 34a and 34b to provide guide wire 10 with a smooth, roughly continuous delivery profile. Coil 38 may be connected to distal tip 30 by a variety of techniques, per se known, including laser welding and/or adhesives, e.g. photocurable adhesives. Coil 38 is preferably fabricated from a radiopaque material, such as gold, platinum, iridium, or a combination thereof.

With reference to FIG. 1C, section 32 of floppy distal tip 30 preferably has a flat profile, i.e. a rectangular cross-section. The rectangular cross-section, in conjunction with the decreased length 1 of tip 30, is expected to cause the distal tip to vibrate, or "dance", when positioned within a blood jet at the outlet of a heart valve. This vibratory motion may be imaged and may be used to indicate when the distal tip is properly positioned within the blood jet. When so positioned, the guide wire may be advanced through the heart valve without damaging the leaflets of the valve or, when the valve is stenosed, generating emboli. The radiopacity of coil 38 is expected to facilitate imaging.

Section 32 preferably has a large aspect ratio of height **H** to width **W**. Width **W** preferably is less than or equal to about 0.5mm, and more preferably is less than or equal to about 0.15mm. Height **H**, meanwhile, preferably is greater than or equal to about 2mm, and more preferably is greater than or equal to about 4mm. An upper bound for the height **H** is provided by the internal diameter of coil 38, which preferably is about 6mm. Section 32 may be formed, for example, by flattening that portion of guide wire 10 with a press.

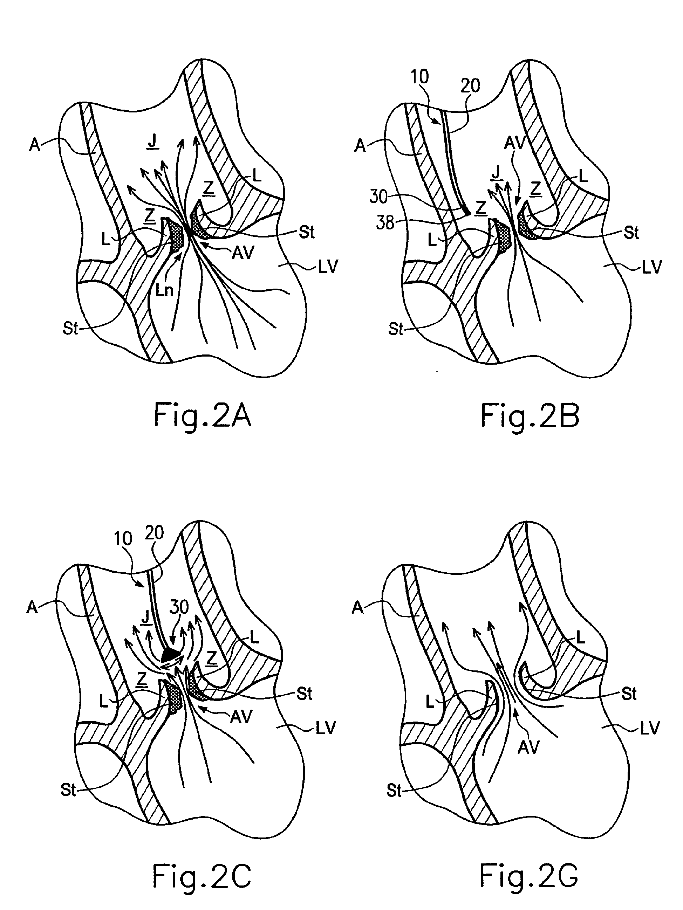

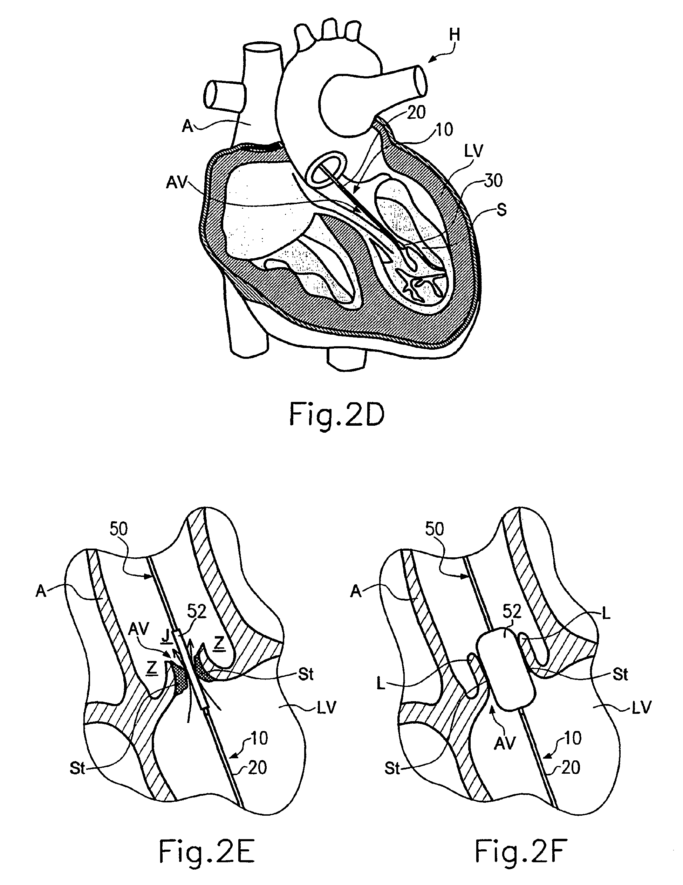

Referring now to FIGS. 2, a method of using guide wire 10 in accordance with the present invention is described. In FIG. 2A, a patient's defective aortic valve **AV**, having leaflets **L**, is shown narrowed with stenosis **St**. The aortic valve regulates blood flow from the patient's left ventricle **LV** into the patient's aorta **A**. Stenosis **St** has reduced the size of lumen **Ln** through the valve and interrupts normal flow between left ventricle **LV** and aorta **A**. Stenosis **St** also may significantly increase the pressure and velocity of blood jet **J** flowing through valve **AV**, while establishing stagnation zones **Z** of decreased velocity on either side of the narrowed outlet of aortic valve **AV**. The increased velocity of blood within jet **J** may make it difficult or impractical to pass a standard guide wire, and therefore additional catheters, across the valve without damaging leaflets **L** or generating emboli from contact with stenosis **St**.

As seen in FIG. 2B, guide wire 10 has been advanced to a position within aorta **A** just proximal of aortic valve **AV**, using well-known percutaneous techniques. For example, floppy distal tip 30 of guide wire 10 has been inserted into the patient's femoral artery through a small puncture near his or her groin. Guide wire 10 then has been advanced to the outlet of valve **AV** under angiographic and/or fluoroscopic visualization. The radiopacity of coil 38 of distal tip 30 facilitates fluoroscopic visualization.

In FIG. 2B, floppy tip 30 vibrates or dances only minimally during the systole phase of the cardiac cycle, because it is located in low velocity stagnation zone **Z**, where blood flow only minimally deflects relatively floppy tip 30. Guide wire 10 thus is repositioned such that distal tip 30 is located within blood jet **J** exiting aortic valve **AV**, as in FIG. 2C. It is expected that a physician will be able to determine when floppy tip 30 is located within jet **J** due to vibration of tip 30 during systole, as can be visualized, for example, by imaging radiopaque coil 38 with a fluoroscope. The use of alternative or additional imaging techniques, including magnetic resonance imaging ("MRI"), intravascular ultrasound ("IVUS"), and optical coherence tomography ("OCT"), will be apparent to those of skill in the art.

Once floppy distal tip 30 is positioned within jet J, the physician is able to synchronize insertion of wire 10 into left ventricle **LV** with the systole phase of the cardiac cycle, in which leaflets **L** of aortic valve **AV** are open, since vibration of distal tip 30 is expected to decrease substantially or completely during the diastole phase, wherein leaflets **L** are closed. This reduces a risk of damage to the leaflets from crossing/insertion attempts during diastole, as well as a risk of emboli generation due to contact of distal tip 30 of wire 10 with stenosis **St**. Even if insertion of wire 10 is inadvertently attempted during diastole, the floppiness of distal tip 30, as compared to the floppiness of standard wires that currently may be used, is expected to deflect tip 30 away from leaflets **L**, thereby reducing trauma to the leaflets and reducing emboli generation. Wire 10 then may be repositioned in jet **J**, and crossing may be reattempted during systole.

In FIG. 2D, guide wire 10 has been advanced across stenosed aortic valve **AV** into left ventricle **LV** of heart **H**. If wire 10 is inadvertently over-advanced into left ventricle **LV**, the floppiness of distal tip 30 is expected to deflect tip 30 away from the ventricular wall, and thereby reduce a risk of myocardial perforation, as compared to standard guide wires. Guide wire 10 illustratively is shown with optional sensor **S** in FIG. 2D. Sensor **S** may comprise, for example, a pressure sensor, so that transstenotic/transvalvular pressure loss may be quantified. This pressure loss then may be used to determine if the patient is a candidate for valvular repair, for example, via valvuloplasty, or if more invasive valvular replacement is required. Alternatively, additional sensors and/or catheters (not shown) may be advanced over wire 10 and across aortic valve **AV** for imaging and/or diagnostic measurements, so that the best course of treatment may be determined.

In FIG. 2E, it has been determined that valvuloplasty is the best treatment modality for stenosed aortic valve **AV**. Catheter 50 having valvuloplasty balloon 52 disposed in a collapsed delivery configuration is advanced over wire 10 to a position wherein balloon 52 is disposed across aortic valve **AV**. Balloon 52 is expanded to a deployed configuration, as seen in FIG. 2F, to compress stenosis **St**, widen the valve, and improve flow through the valve. The balloon may be expanded and collapsed several times to stretch the hardened valve. Heart measurements may be taken to ensure that valve function has improved. Catheter 50 and guide wire 10 then are removed from the patient, and more normal flow proceeds through aortic valve **AV,** as seen in FIG. 2G.

In FIGS. 2, imaging vibration of distal tip 30 of guide wire 10 facilitates crossing of the aortic valve. However, it should be understood that tip 30 may not vibrate in every patient, or may vibrate only minimally, It is expected that the presence and/or extent of vibration will be affected by a variety of factors, including the velocity and volume of blood flowing through the valve.

Although preferred illustrative embodiments of the present invention are described hereinabove, it will be evident to those skilled in the art that various changes and modifications may be made therein without departing from the invention. For example, in FIGS. 2, guide wire 10 illustratively is shown in use at a stenosed aortic valve, but it should be understood that guide wire 10 also may be used at a healthy valve, at a regurgitating valve, or at an otherwise defective valve, In addition to being used at a stenotic valve. Likewise, guide wire 10 may be used in the tricuspid, mitral, and pulmonary heart valves, in addition to the aortic valve - as well as in diagnostic and therapeutic procedures at other places within the patient's vasculature, or within other body lumens. It is expected, for example, that guide wires of the present invention will have particular utility in crossing stenosed and/or tortuous arterial segments within patients suffering from peripheral artery disease. The wires also may be used to partially or completely straighten curved arterial segments, prior to treatment of the segments via balloon angioplasty, stent placement, brachytherapy, etc.

## Claims

1. A guide wire (10) adapted for crossing a heart valve, the guide wire (10) comprising:
- a core wire (20); and
- a floppy distal tip (30) coupled to the core wire (20), wherein
- the floppy tip (30) comprises a section (32) of reduced cross-sectional area, the section being connected on either side to transition sections (34a, 34b) that taper from the cross section of the core wire (20) to the cross section of section (32).

2. The guide wire of claim 1, wherein the floppy distal tip (30) has a longitudinal length less than about 5 cm.

3. The guide wire of claim 1, wherein the core wire (20) has an outer diameter of at least about 0,6 mm.

4. The guide wire of claim 1, wherein the floppy distal tip (30) has a longitudinal length less than or equal to about 2,5 cm.

5. The guide wire of claim 1, wherein the core wire (20) has a maximum outer diameter of at least about 0,69 mm.

6. The guide wire of claim 1, wherein the guide wire (20) has a length less than or equal to about 200 cm.

7. The guide wire of claim 6, wherein the guide wire (20) has a length less than or equal to about 150 cm.

8. The guide wire of claim 1, wherein the section (32) of reduced cross-sectional area has a flat profile.

9. The guide wire of claim 1, wherein the section (32) of reduced cross-sectional area has a rectangular cross-section.

10. The guide wire of claim 1, wherein the floppy distal tip (30) further comprises a coil (38) disposed about the section (32) of reduced cross-sectional area.

11. The guide wire of claim 10, wherein the coil (38) is radiopaque.

12. The guide wire of claim 1 further comprising a coating disposed about the guide wire.

13. The guide wire of claim 12, wherein the coating is chosen from the group consisting of hydrophilic coatings, polytetrafluoroethylene coatings, therapeutic drug coatings, and anti-thrombotic coatings.

14. The guide wire of claim 1 further comprising a diagnostic sensor coupled to the guide wire (10).

15. The guide wire of claim 14, wherein the diagnostic sensor is chosen from the group consisting of flow sensors, pressure sensors, temperature sensors, and imaging sensors.

16. The guide wire of claim 1, wherein a distal bulb (36) is connected to the transition section (34a), being distally from the reduced cross-sectional area.

## Patentansprüche

1. Führungsdraht (10) zum Durchdringen einer Herzklappe, wobei der Führungsdraht (10) umfasst:
- einen Kerndraht (20); und
- eine flexible distale Spitze (30), die mit dem Kerndraht (20) verbunden ist, wobei
- die flexible Spitze (30) einen Abschnitt (32) mit einem verringerten Querschnittsbereich aufweist, wobei der Abschnitt auf jeder Seite mit Übergangsabschnitten (34a, 34b) verbunden ist, die sich vom Querschnitt des Kerndrahts (20) zum Querschnitt des Abschnitts (32) verjüngen.

2. Führungsdraht nach Anspruch 1, wobei die flexible distale Spitze (30) eine Länge in Längsrichtung von weniger als ca. 5 cm aufweist.

3. Führungsdraht nach Anspruch 1, wobei der Kerndraht (20) einen Außendurchmesser von zumindest ca. 0,6 mm aufweist.

4. Führungsdraht nach Anspruch 1, wobei die flexible distale Spitze (30) eine Länge in Längsrichtung von weniger als oder gleich ca. 2,5 cm aufweist.

5. Führungsdraht nach Anspruch 1, wobei der Kerndraht (20) einen maximalen Außendurchmesser von zumindest ca. 0,69 mm aufweist.

6. Führungsdraht nach Anspruch 1, wobei der Führungsdraht (20) eine Länge von weniger als oder gleich ca. 200 cm aufweist.

7. Führungsdraht nach Anspruch 6, wobei der Führungsdraht (20) eine Länge von weniger als oder gleich ca. 150 cm aufweist.

8. Führungsdraht nach Anspruch 1, wobei der Abschnitt (32) mit verringertem Querschnittsbereich ein flaches Profil aufweist.

9. Führungsdraht nach Anspruch 1, wobei der Abschnitt (32) mit verringertem Querschnittsbereich einen rechteckigen Querschnitt aufweist.

10. Führungsdraht nach Anspruch 1, wobei die flexible distale Spitze (30) ferner eine Spule (38) aufweist, die um den Abschnitt (32) mit verringertem Querschnittsbereich angeordnet ist.

11. Führungsdraht nach Anspruch 10, wobei die Spule (38) röntgendicht ist.

12. Führungsdraht nach Anspruch 1, ferner umfassend eine Beschichtung, die um den Führungsdraht angeordnet ist.

13. Führungsdraht nach Anspruch 12, wobei die Beschichtung aus der Gruppe bestehend aus hydrophilen Beschichtungen, Polytetrafluorethylen-Beschichtungen, Beschichtungen aus therapeutischen Arzneimitteln und Antithrombose-Beschichtungen ausgewählt ist.

14. Führungsdraht nach Anspruch 1, ferner umfassend einen Diagnosesensor, der mit dem Führungsdraht (10) verbunden ist.

15. Führungsdraht nach Anspruch 14, wobei der Diagnosesensor aus der Gruppe bestehend aus Durchfluss-Sensoren, Drucksensoren, Temperatursensoren und bildgebenden Sensoren ausgewählt ist.

16. Führungsdraht nach Anspruch 1, wobei eine distale Küvette (36) mit dem Übergangsabschnitt (34a), der distal vom verringerten Querschnittsbereich liegt, verbunden ist.

## Revendications

1. Fil guide (10) adapté pour traverser une valvule cardiaque, le fil guide (10) comprenant :
- un fil central (20) ; et
- une extrémité distale souple (30) couplée au fil central (20), dans lequel
- l'extrémité souple (30) comprend une section (32) d'aire de coupe transversale réduite, la section étant connectée de chaque côté aux sections de transition (34a, 34b) qui diminuent progressivement de la coupe transversale du fil central (20) à la coupe transversale de la section (32).

2. Fil guide selon la revendication 1, dans lequel l'extrémité distale souple (30) a une longueur longitudinale inférieure à environ 5 cm.

3. Fil guide selon la revendication 1, dans lequel le fil central (20) a un diamètre externe d'au moins 0,6 mm environ.

4. Fil guide selon la revendication 1, dans lequel l'extrémité distale souple (30) a une longueur longitudinale inférieure ou égale à environ 2,5 cm.

5. Fil guide selon la revendication 1, dans lequel le fil central (20) a un diamètre externe maximal d'au moins 0,69 mm environ.

6. Fil guide selon la revendication 1, dans lequel le fil guide (20) a une longueur inférieure ou égale à environ 200 cm.

7. Fil guide selon la revendication 6, dans lequel le fil guide (20) a une longueur inférieure ou égale à environ 150 cm.

8. Fil guide selon la revendication 1, dans lequel la section (32) de l'aire de coupe transversale réduite a un profil plat.

9. Fil guide selon la revendication 1, dans lequel la section (32) de l'aire de coupe transversale réduite a une coupe transversale rectangulaire.

10. Fil guide selon la revendication 1, dans lequel l'extrémité distale souple (30) comprend en outre une spirale (38) disposée autour de la section (32) de l'aire de coupe transversale réduite.

11. Fil guide selon la revendication 10, dans lequel la spirale (38) est radio-opaque.

12. Fil guide selon la revendication 1, comprenant en outre un revêtement disposé autour du fil guide.

13. Fil guide selon la revendication 12, dans lequel le revêtement est choisi dans le groupe constitué par les revêtements hydrophiles, les revêtements polytétrafluoroéthylènes, les revêtements thérapeutiques médicamenteux et les revêtements antithrombotiques.

14. Fil guide selon la revendication 1, comprenant un capteur de diagnostic couplé au fil guide (10).

15. Fil guide selon la revendication 14, dans lequel le capteur de diagnostic est choisi dans le groupe constitué par les capteurs de flux, les capteurs de pression, les capteurs de température et les capteurs d'images.

16. Fil guide selon la revendication 1, dans lequel un bulbe distal (36) est connecté à la section de transition (34a), à distance de l'aire de coupe transversale réduite.
